# EUROPEAN PATENT APPLICATION

(11) **EP 3 135 686 A1**
(43) Date of publication of application: **01.03.2017**
(21) Application number: 15182840.7
(22) Date of filing: 28.08.2015
(51) Int. Cl.: C07K 14/61, A61K 38/00

(54) **NOVEL RECOMBINANT CYCLIZED HUMAN GROWTH HORMONE**

(71) Applicant: Latvian Biomedical Research and Study Centre, 1067 Riga (LV)
(72) Inventor: RUDEVICA, Zhanna, LV-1067 Riga (LV); BOGANS, Janis, LV-1067 Riga (LV); ZHULENKOVS, Dmitrijs, LV-1067 Riga (LV); KURBATSKA, Viktorija, LV-1067 Riga (LV); LEONCIKS, Ainars, LV-1067 Riga (LV)
(74) Representative: Kuzjukevica, Lucija

(57) **Abstract**

The present invention relates to a novel recombinant cyclized human growth hormone (somatotropin) which can be used in medicine for treating conditions associated with growth hormone deficiency.

## Description

### Subject of invention

The present invention relates to a novel recombinant human growth hormone (somatotropin), more particularly to a novel recombinant cyclized human somatotropin. The new recombinant cyclized human growth hormone (rc-hGH) can be used in medicine for treating conditions associated with growth hormone deficiency.

### Background art

Somatotropin or growth hormone (GH) is a peptide hormone, mainly produced in somatotropic cells within the lateral wings of the anterior pituitary gland. GH is a hormone that stimulates muscle and bone growth, regulates carbohydrate and lipid metabolism as well as cardiac activity. Its deficiency causes different pathologies. GH is used as replacement therapy in patients with growth hormone deficiency of either childhood-onset or adult-onset (Blizzard RM. History of growth hormone therapy. Indian J Pediatr. 2012;79:87-91).

The major isoform of human growth hormone is a protein of 191 amino acids, and contains two disulfide bonds. Somatotropin has been produced by recombinant DNA techniques. Although hGH is widely accepted therapeutical agent, its clinical use has some disadvantages, such as need of frequent injections due to short degradation time in plasma (Takeda A, et al. Recombinant human growth hormone for the treatment of growth disorders in children: a systematic review and economic evaluation Health Technol Assess. 2010; 14:1-209).

To prolong hGH activity, the protein modification such as covalent binding to polyethylene glycol (PEG) is used. Although this method allows to increase protein stability, it includes modifications of purified GH and therefore separation of active preparation and reaction byproducts leading to remarkable loss of end product (da Silva FD, et al. Chemical and enzymatic site specific PEGylation of hGH. Bioconjugate Chem. 2013; 24:456-63).

Another strategy is to exploit GH containing particles of biodegradable polymer. This approach is technologically sophisticated and costly (Kim HK, Park TG. Microencapsulation of human growth hormone within biodegradable polyester microspheres: protein aggregation stability and incomplete release mechanism. Biotechnol Bioeng. 1999; 65:659-67).

Optimization of hGH production as therapeutic entity would benefit the treatment of GH deficiencies, and reduce the discomfort and inconvenience associated with known hGH medications It would provide a pharmaceutical agent that would be more stable and could be administered less frequently.

Increased protein stability can be achieved by cyclization, when termini of linear protein molecules are joined by covalent bound. Since cyclic polypeptides cannot be cleaved by exopeptidases, cyclic molecules are more stable in the presence of proteases [Bankowski K, et al. Synthesis, biological activity and resistance to proteolytic digestion of new cyclic dermorphin/deltorphin analogues. Eur J Med Chem. 2013;63:457-67; Molhoek EM, et al. Improved proteolytic stability of chicken cathelicidin-2 derived peptides by D-amino acid substitutions and cyclization. Peptides. 2011; 32:875-80.] and have more stable spatial structure. Therefore, these molecules maintain their characteristics even in unfavourable conditions such as high temperature or denaturizing environment [Iwai H, et al. Cyclic green fluorescent protein produced in vivo using and artificially Split PI-fuI intein from Pyrococcus furiosus. J. Biol. Chem. 2001; 276:16548-16554.; Takahashi H, et al. Stabilization of hyperactive dihydrofolate reductase by cyanocysteine-mediated backbone cyclization. J Biol Chem. 2007; 282:9420-9].

In order to circulize protein, cysteines forming disulfide bonds can be inserted into protein sequence [Takahashi H, et al. Stabilization of hyperactive dihydrofolate reductase by cyanocysteine-mediated backbone cyclization. J Biol Chem. 2007; 282:9420-9]. However, cysteine insertion may negatively affect protein structure and characteristics and disulfide bonds are sensitive to reductants.

Post-translational modification, termed intein-mediated protein cyclization (circularization) where the NH₂- and COOH-terminal ends of normally linear proteins are cross-linked, offers an attractive approach for the protein thermodynamic stabilization and protection against cellular catabolism, described in [Iwai H, Plückthun A. Circular beta-lactamase: stability enhancement by cyclizing the backbone. FEBS Lett. 1999; 459:166-72. Scott CP, et al. Production of cyclic peptides and proteins in vivo. Proc Natl Acad Sci USA 1999; 96:13638-43]. Intein domains are responsible for posttranslational modification and are expressed with target protein (extein). Immediately after translation inteins autocatalyze self-cleavage reaction [Perler FB, et al. Protein splicing elements: inteins and exteins - a definition of terms and recommended nomenclature. Nucleic Acids Res. 1994; 22: 1125-7]. Beside of self-cleavage, posttranslational splicing mechanism includes formation of peptide bonds between extein amino acids [Ding Y, et al. Crystal structure of a mini-intein reveals a conserved catalytic module involved in side chain cyclization of asparagine during protein splicing. J.Biol.Chem. 2003; 278:39133-42].

This method of protein covalent cyclization is of special interest since it can be used to enhance the stability of various enzymes, in particular in pharmaceutical applications. Herein, we describe the use of a synthetic *Synechocystis sp.* PCC6803 mini-intein gene, to cyclize a human somatotropin protein *in vivo* described in paper of Williams (Williams NK, et al. In Vivo Protein Cyclization Promoted by a Circularly Permuted Synechocystis sp. CC6803 DnaB Mini-intein. J Biol Chem. 2002; 277:7790-8). Since intein self-cleavage is autocatalytic process, precursor splicing and extein cyclization occur immediately after translation *in vivo.* Therefore, it is possible to express circular target protein directly without chemical manipulations. Recombinant hGH can be expressed in *E. coli,* and protocol for high protein yields was described (Kim MJ, et al. Complete Solubilization and Purification of Recombinant Human Growth Hormone Produced in Escherichia coli. PLoS One. 2013; 8:e561-68).

Moreover, additional sequence of 18 amino acids with high affinity to blood plasma albumin was inserted in front of N-terminus of hGH. This sequence is supposed to extend half-life of the target protein by non-covalent association with albumin and increase solubility of hGH necessary for protein cyclization in *E.coli* expression system (Dennis MS, et al. Albumin binding as a general strategy for improving the pharmacokinetics of proteins. J Biol Chem. 2002; 277:35035-43).

We have disclosed absolutely new product - recombinant cyclized human growth hormone.

### Summary of invention

The present invention discloses a novel recombinant cyclized human growth hormone. The cyclization of somatotropin is achieved by trans-splicing reaction catalysed by *Synechocystis sp.* PCC6803 intein where the N and C termini of the target protein is linked by a normal peptide bond, which results in much higher stability of the molecule in comparison to the linear prototype.

Here is provided a synthesized nucleic acid molecule encoding the codon optimized human growth hormone with albumin binding peptide (ABP; in italic) of the present invention, including mRNA, cDNA and DNA as well as analogues and derivatives thereof (SEQ ID No. 1):
5'-*CGCCTGATTGAAGACATTTGCCTGCCGCGCTGGGGCTGCCTGTGGGAAGACGAC* TTTCCGACGATTCCGCTGTCTCGTCTGTTTGATAACGCGATGCTGCGTGCACA TCGTCTGCACCAGCTGGCGTTTGACACCTATCAAGAATTTGAAGAAGCCTAC ATTCCGAAAGAACAGAAATATAGCTTTCTGCAGAATCCGCAAACGTCTCTGT GCTTCAGCGAATCTATCCCGACCCCGAGTAACCGTGAAGAAACGCAGCAGAA AAGCAATCTGGAACTGCTGCGCATTAGTCTGCTGCTGATCCAGTCCTGGCTG GAACCGGTCCAATTTCTGCGTAGCGTGTTCGCAAACTCTCTGGTTTACGGTGC TAGTGATTCCAATGTCTATGATCTGCTGAAAGACCTGGAAGAAGGCATTCAG ACCCTGATGGGCCGTCTGGAAGATGGTAGCCCGCGCACGGGCCAGATCTTTA AACAAACCTACTCAAAATTCGACACGAACTCGCATAATGATGACGCGCTGCT GAAAAACTATGGTCTGCTGTACTGTTTTCGCAAAGATATGGACAAAGTGGAA ACCTTCCTGCGTATCGTGCAGTGCCGCTCAGTTGAAGGCTCGTGTGGTTTC-3' adapted for expression in *E.coli* and cloned in the appropriate vector for further DNA manipulations (linear recombinant hGH).

The PCR amplified ABP-hGH sequence (SEQ ID No.1) with *EcoRI* and *MluI* cleavage sites was cloned between C-intein (Int_{C}) and N-intein (Int_{N}) domains in the vector with IPTG-inducible phage T7-promoter [Williams NK, et al. J Biol Chem. 2002; 277:7790-8]. The expression of the resulting Int_{C}-ABP-hGH-Int_{N} fusion protein subsequently leads to generation of a recombinant cyclized human growth hormone (rc-hGH) in a trans-splicing reaction catalysed by both mini-inteins domains. The resulting rc-hGH protein contained additional 9 amino acid sequence SIEFTRESG inserted between the N- and C-termini of somatotropin that stand for the Int_{C} (SIEF) and Int_{N} (TRESG) encoding fragments of the split mini-intein gene of the Int_{C}-ABP-hGH-Int_{N} fusion protein.

A full DNA sequence of the Int_{C}-ABP-hGH-Int_{N} fusion molecule (SEQ ID No. 2) is provided here:
5'-atgtctccggaaatcgaaaaactgtctcagtctgacatttactgggactctattgtgtctattaccgaaaccggtgtagaagaagttt *tcgacctgaccgtgccaggaccgcacaacttcgtggccaacgacatcatcgttcacaactctatcgaattcCGCCTGATT GAAGACATTTGCCTGCCGCGCTGGGGCTGCCTGTGGGAAGACGAC*ATGGCGACG GGCTCTCGTACCTCACTGCTGCTGGCGTTTGGTCTGCTGTGTCTGCCGTGGCT GCAAGAAGGCTCCGCATTTCCGACGATTCCGCTGTCTCGTCTGTTTGATAACG CGATGCTGCGTGCACATCGTCTGCACCAGCTGGCGTTTGACACCTATCAAGA ATTTGAAGAAGCCTACATTCCGAAAGAACAGAAATATAGCTTTCTGCAGAAT CCGCAAACGTCTCTGTGCTTCAGCGAATCTATCCCGACCCCGAGTAACCGTG AAGAAACGCAGCAGAAAAGCAATCTGGAACTGCTGCGCATTAGTCTGCTGCT GATCCAGTCCTGGCTGGAACCGGTCCAATTTCTGCGTAGCGTGTTCGCAAACT CTCTGGTTTACGGTGCTAGTGATTCCAATGTCTATGATCTGCTGAAAGACCTG GAAGAAGGCATTCAGACCCTGATGGGCCGTCTGGAAGATGGTAGCCCGCGC ACGGGCCAGATCTTTAAACAAACCTACTCAAAATTCGACACGAACTCGCATA ATGATGACGCGCTGCTGAAAAACTATGGTCTGCTGTACTGTTTTCGCAAAGA TATGGACAAAGTGGAAACCTTCCTGCGTATCGTGCAGTGCCGCTCAGTTGAA GGCTCGTGTGGTTTCacgcgtgagtctggctgcatctctggtgattctctgatcagcctggcgagcaccggtaaac gtgtttctatcaaagatctgctggacgaaaaagatttcgaaatctgggcaatcaacgaacagaccatgaaactggaatctgctaaa gtttctcgtgtgttctctactggtaaaaaactggtttacattctgaaaactcgactgggtcgtaccatcaaagcgaccgcgaatcac cgtttcctgactatcgacggttggaaacgtctggatgagctgtctctgaaagaacatattgctctgccgcgtaaactggagagctc ctctctgcagctgtaa-3', where the intein cassette sequences are marked with small letters, ABP sequence is marked in italic, and somatotropin gene sequence is marked with capital letters.

The present invention is above mentioned recombinant cyclized human growth hormone (rc-hGH) with the amino acid sequence that comprises the amino acid sequence coded by DNA sequence given in SEQ ID No. 1. The recombinant cyclized human growth hormone further comprises the amino acid sequence coded by DNA sequence given in SEQ ID No. 2 Said recombinant cyclized human growth hormone can be used in a method for the treatment of growth hormone deficiency. The recombinant human cyclized somatotropin can be used in the treatment of conditions associated with growth hormone deficiency. Use of a recombinant cyclized human growth hormone for the manufacture of a medicament for administration simultaneously, sequentially or separately for treatment of conditions associated with growth hormone deficiency.

### Brief description of the drawings

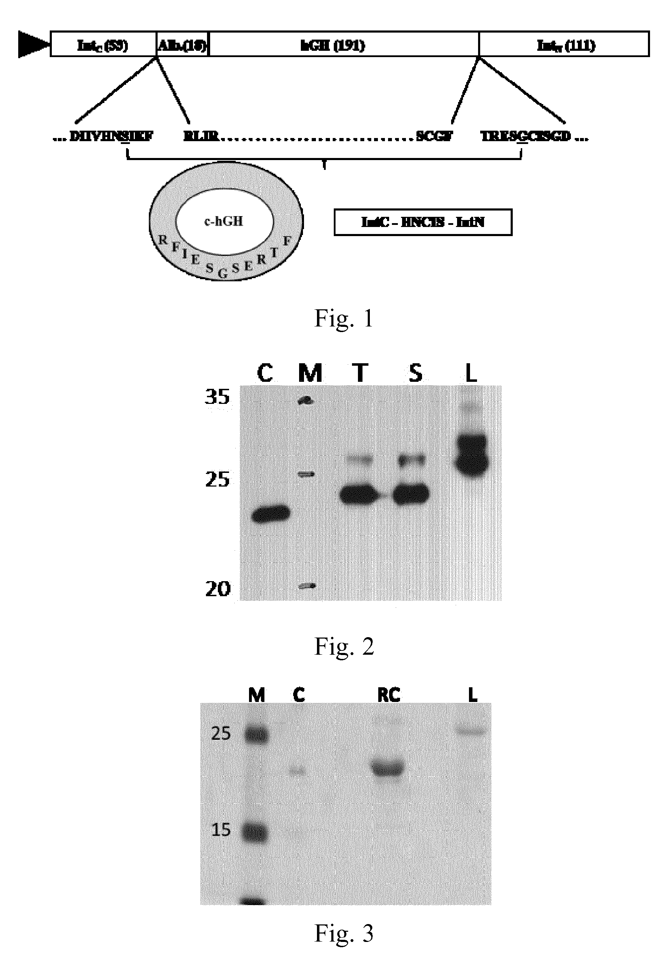

Fig. 1 represents the strategy used for somatotropin cyclization, namely the split mini-intein trans-splicing technology described by Williams NK (Williams NK, et al. In vivo Protein Cyclization Promoted by a Circularly Permuted Synechocystis sp. CC6803 DnaB Mini-intein. J Biol Chem. 2002; 277:7790-8).
Fig. 2 represents Western Blot analysis of rc-hGH expression after induction with IPTG, using anti-hGH antibodies. Cell lysate is separated in 15% SDS-PAGE. C - commercial growth hormone; M - marker, kDa; T - rc-hGH, total E.coli cell lysate; S - rc-hGH, soluble protein fraction; L - linear r-hGH.
Fig. 3 illustrates purified rc-hGH polypeptide after size-exclusion chromatography.
Samples were separated in 15% SDS-PAGE and stained with Coomassie Blue. C - commercial growth hormone; M - marker, kDa; RC - rc-hGH; L - linear r-hGH.

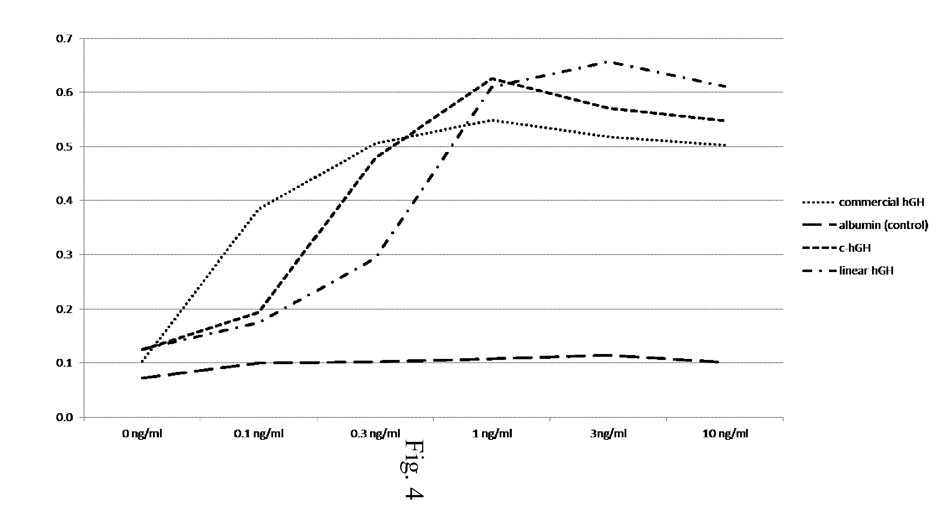

Fig. 4 shows the results of *in vitro* assay for rc-hGH activity. *In vitro* biological activity of recombinant human cyclized somatotropin was evaluated according to the colorimetric MTT cell viability assay using cell line Nb2-11 that proliferates only in the presence of somatotropin in the culture medium as it was described in paper of Gout PW (Gout PW, et al. Prolactin-stimulated growth of cell cultures established from malignant Nb rat lymphomas. Cancer Res. 1980; 40:2433-2436).

The present invention relates to bacterial production of modified hGH polypeptide (Fig.1) as an alternative and a more economical option for synthesis of biologically active product.

Synthesis of recombinant cyclic hGH polypeptides was achieved by the method of bacterial expression system where Escherichia coli BL21(DE3) or other suitable strain was transformed with modified pET system vector (Novagen) that express the IntCABP-hGH-IntN sequence under the control of an IPTG-inducible T7 promoter. Transformants inoculated with E.coli harbouring recombinant plasmids were grown at 37°C on Luria-Bertani (LB) agar supplemented with ampicillin (50 mg/litre) to select for the rc-hGH construct. LB broth, supplemented with 0.1% glucose and ampicillin. The transformed strain could be grown at 37°C until A₆₀₀=0.6-0.7, and then IPTG was added to a final concentration of 1mM and the culture was incubated at 15°C for 16 hours. The cells were harvested by centrifugation, re-suspended in sonication buffer containing a complete protease inhibitor cocktail (Roche Diagnostics AG) and lysed by sonication. The linear and cyclized ABP-hGH polypeptides were analysed by Western immunoblotting using goat polyclonal hGH antibody A-18 (Santa Cruz Biotechnology, Inc). The results are represented in Fig. 2.

The synthesized rc-hGH protein was purified by size-exclusion chromatography on three Superdex 200 columns (GE Healthcare Life Sciences) connected in series. The purified rc-hGH was visualized by Coomassie Blue staining after SDS-PAGE separation. The results are represented in Fig. 3. In addition, the recombinant c-hGH was purified from bacterial endotoxins by Detoxi-Gel endotoxin-removing gel column (Thermo Fisher Scientific Inc.). Molecular weight of rc-hGH was estimated by mass spectroscopy (Bruker Corp.).

The biological activity of purified rc-hGH was evaluated *in vitro* by assessing its growth promoting action using the prolactin-dependent rat lymphoma Nb2-11 cell line (Gout PW, et al. Prolactin-stimulated growth of cell cultures established from malignant Nb rat lymphomas. Cancer Res. 1980; 40:2433-2436). Commercially available recombinant hGH expressed in *E.coli* (Sigma-Aldrich) was used as a control in the assay. 10⁴ cells seeded into 96-well flat-bottom culture plates were grown in 100µl of culture medium and in the presence of different concentrations of commercial hGH, albumin (BSA), cyclic hGH and linear hGH. Cells were incubated for 72 hours in cell culture incubator in 5% CO₂ at 37°C temperature and MTT solution was added to each well. The plates were read at 540 nm in F200 reader (Tecan Group Ltd.) after complete solubilisation of the dye. The results presented in Figure 4 were expressed as cell number (OD value) vs hGH concentration in the medium according to the colorimetric MTT cell viability assay (Vybrant MTT Cell Proliferation Assay Kit, Invitrogen Corp.). All experiments were done in triplicate, and repeated three times. Our results indicated that the rc-hGH was biologically active.

Sequence listings

<Sequence ID number 210> 1
<Sequence length 211> 627
<Molecule type 212> DNA
<Organism 213> Artificial Sequence
<Additional sequence description 223> Selected extein sequence
<Sequence name 400> 1
cgcctgattg aagacatttg cctgccgcgc tggggctgcc tgtgggaaga cgactttccg 60
acgattccgc tgtctcgtct gtttgataac gcgatgctgc gtgcacatcg tctgcaccag 120
ctggcgtttg acacctatca agaatttgaa gaagcctaca ttccgaaaga acagaaatat 180
agctttctgc agaatccgca aacgtctctg tgcttcagcg aatctatccc gaccccgagt 240
aaccgtgaag aaacgcagca gaaaagcaat ctggaactgc tgcgcattag tctgctgctg 300
atccagtcct ggctggaacc ggtccaattt ctgcgtagcg tgttcgcaaa ctctctggtt 360
tacggtgcta gtgattccaa tgtctatgat ctgctgaaag acctggaaga aggcattcag 420
accctgatgg gccgtctgga agatggtagc ccgcgcacgg gccagatctt taaacaaacc 480
tactcaaaat tcgacacgaa ctcgcataat gatgacgcgc tgctgaaaaa ctatggtctg 540
ctgtactgtt ttcgcaaaga tatggacaaa gtggaaacct tcctgcgtat cgtgcagtgc 600
cgctcagttg aaggctcgtg tggtttc 627

< Sequence ID number 210> 2
< Sequence length 211> 1200
< Molecule type 212> DNA
< Organism 213> Artificial Sequence
< Additional sequence description 223> Selected extein sequence
<400> 2
atgtctccgg aaatcgaaaa actgtctcag tctgacattt actgggactc tattgtgtct 60
attaccgaaa ccggtgtaga agaagttttc gacctgaccg tgccaggacc gcacaacttc 120

gtggccaacg acatcatcgt tcacaactct atcgaattcc gcctgattga agacatttgc 180
ctgccgcgct ggggctgcct gtgggaagac gacatggcga cgggctctcg tacctcactg 240
ctgctggcgt ttggtctgct gtgtctgccg tggctgcaag aaggctccgc atttccgacg 300
attccgctgt ctcgtctgtt tgataacgcg atgctgcgtg cacatcgtct gcaccagctg 360
gcgtttgaca cctatcaaga atttgaagaa gcctacattc cgaaagaaca gaaatatagc 420
tttctgcaga atccgcaaac gtctctgtgc ttcagcgaat ctatcccgac cccgagtaac 480

SEQUENCE LISTING

<110> Latvian Biomedical Research and Study Centre

<120> Novel recombinant cyclized human growth hormone

<130> AK/P3125

<160> 2

<170> BiSSAP 1.3

<210> 1
<211> 627
<212> DNA
<213> Artificial Sequence

<220>
<223> Selected extenin sequence

<400> 1
cgcctgattg aagacatttg cctgccgcgc tggggctgcc tgtgggaaga cgactttccg 60

acgattccgc tgtctcgtct gtttgataac gcgatgctgc gtgcacatcg tctgcaccag 120

ctggcgtttg acacctatca agaatttgaa gaagcctaca ttccgaaaga acagaaatat 180

agctttctgc agaatccgca aacgtctctg tgcttcagcg aatctatccc gaccccgagt 240

aaccgtgaag aaacgcagca gaaaagcaat ctggaactgc tgcgcattag tctgctgctg 300

atccagtcct ggctggaacc ggtccaattt ctgcgtagcg tgttcgcaaa ctctctggtt 360

tacggtgcta gtgattccaa tgtctatgat ctgctgaaag acctggaaga aggcattcag 420

accctgatgg gccgtctgga agatggtagc ccgcgcacgg gccagatctt taaacaaacc 480

tactcaaaat tcgacacgaa ctcgcataat gatgacgcgc tgctgaaaaa ctatggtctg 540

ctgtactgtt ttcgcaaaga tatggacaaa gtggaaacct tcctgcgtat cgtgcagtgc 600

cgctcagttg aaggctcgtg tggtttc 627


<210> 2
<211> 1200
<212> DNA
<213> Artificial Sequence

<220>
<223> Selected extenin sequence

<400> 2
atgtctccgg aaatcgaaaa actgtctcag tctgacattt actgggactc tattgtgtct 60

attaccgaaa ccggtgtaga agaagttttc gacctgaccg tgccaggacc gcacaacttc 120

gtggccaacg acatcatcgt tcacaactct atcgaattcc gcctgattga agacatttgc 180

ctgccgcgct ggggctgcct gtgggaagac gacatggcga cgggctctcg tacctcactg 240

ctgctggcgt ttggtctgct gtgtctgccg tggctgcaag aaggctccgc atttccgacg 300

attccgctgt ctcgtctgtt tgataacgcg atgctgcgtg cacatcgtct gcaccagctg 360

gcgtttgaca cctatcaaga atttgaagaa gcctacattc cgaaagaaca gaaatatagc 420

tttctgcaga atccgcaaac gtctctgtgc ttcagcgaat ctatcccgac cccgagtaac 480

cgtgaagaaa cgcagcagaa aagcaatctg gaactgctgc gcattagtct gctgctgatc 540

cagtcctggc tggaaccggt ccaatttctg cgtagcgtgt tcgcaaactc tctggtttac 600

ggtgctagtg attccaatgt ctatgatctg ctgaaagacc tggaagaagg cattcagacc 660

ctgatgggcc gtctggaaga tggtagcccg cgcacgggcc agatctttaa acaaacctac 720

tcaaaattcg acacgaactc gcataatgat gacgcgctgc tgaaaaacta tggtctgctg 780

tactgttttc gcaaagatat ggacaaagtg gaaaccttcc tgcgtatcgt gcagtgccgc 840

tcagttgaag gctcgtgtgg tttcacgcgt gagtctggct gcatctctgg tgattctctg 900

atcagcctgg cgagcaccgg taaacgtgtt tctatcaaag atctgctgga cgaaaaagat 960

ttcgaaatct gggcaatcaa cgaacagacc atgaaactgg aatctgctaa agtttctcgt 1020

gtgttctcta ctggtaaaaa actggtttac attctgaaaa ctcgactggg tcgtaccatc 1080

aaagcgaccg cgaatcaccg tttcctgact atcgacggtt ggaaacgtct ggatgagctg 1140

tctctgaaag aacatattgc tctgccgcgt aaactggaga gctcctctct gcagctgtaa 1200

## Claims

1. A recombinant cyclized human growth hormone (rc-hGH) with the amino acid sequence that comprises the amino acid sequence coded by DNA sequence given in SEQ ID No. 1.

2. The recombinant cyclized human growth hormone according to Claim 1 further comprises the amino acid sequence coded by DNA sequence given in SEQ ID No. 2

3. The recombinant cyclized human growth hormone according to Claim 1 or 2 for use in a method for the treatment of growth hormone deficiency.

4. The recombinant human cyclized somatotropin according to claim 1 or 2 for use in the treatment of conditions associated with growth hormone deficiency.

5. Use of a recombinant cyclized human growth hormone of claim 1 or 2 for the manufacture of a medicament for administration simultaneously, sequentially or separately for treatment of conditions associated with growth hormone deficiency.
